# EUROPÄISCHE PATENTANMELDUNG

(11) **EP 0 652 435 A2**
(43) Veröffentlichungstag der Anmeldung: **10.05.1995**
(21) Anmeldenummer: 94116919.5
(22) Anmeldetag: 26.10.1994
(51) Int. Cl.: G01N 33/00, G01N 27/12

(54) **Sensor zur Bestimmung des Verlaufs der Konzentration**

(30) Priorität: 04.11.1993 DE 4337663
(71) Anmelder: SIEMENS AKTIENGESELLSCHAFT, D-80333 München (DE)
(72) Erfinder: Schmelz, Helmut, Dr., D-83209 Prien (DE)

(57) **Zusammenfassung**

Bei der katalytischen Umsetzung von mindestens zwei Reaktanten eines Strömungsmediums ist es zur Erreichung hoher Abscheidegrade bei gleichzeitig geringem Schlupf der Reaktanten erforderlich, die Reaktanten in einem geeigneten stöchiometrischen Verhältnis zueinander mit einem Katalysator in Kontakt zu bringen. Eine Messung der Konzentration der Reaktanten, wie z. B. NH₃ und NOₓ, im Strömungsmedium ist besonders für die genannten Beispiele technisch aufwendig und teuer.

Zur Behebung dieses Mangels ist erfindungsgemäß ein Sensor (4, 46) zur Bestimmung des Verlaufs der Konzentration eines aus einem Strömungsmedium (36) heraus durch eine Oberfläche (33) eines Sensormaterials (34, 54) hindruch in das Sensormaterial (34, 54) eindiffundierenden, adsorbierbaren Stoffes (NH₃) in Abhängigkeit vom Abstand von dieser Oberfläche (33) vorgesehen, bei dem in dem Sensormaterial (34) und gegebenenfalls auf der Oberfläche (33) des Sensormaterials (34, 54) mit elektrischen Zuleitungen (16, 18, 58) versehene Kontaktpaare (12, 62) vorgesehen sind, mittels derer die elektrische Leitfähigkeit des Sensormaterials (34,54) zwischen den Kontakten (28, 30, 62) der einzelnen Kontaktpaare bestimmbar ist. Durch die Messung des Verlaufes der elektrischen Leitfähigkeit in Abhängigkeit von der Materialtiefe sind Rückschlüsse auf die Konzentration des am Sensormaterial adsorbierten Stoffes und auf dessen Konzentration im Strömungsmedium möglich.

Der Sensor eignet sich besonders zur Unterstützung der Abscheidung von Stickoxiden aus Abgasen von Diesel- und Magermotoren.

## Beschreibung

Die Erfindung bezieht sich auf einen Sensor zur Bestimmung des Verlaufs der Konzentration eines aus einem Strömungsmedium heraus durch eine Oberfläche eines Sensormaterials hindurch in das Sensormaterial eindiffundierenden, adsorbierbaren Stoffes, wie z. B. Ammoniak, in Abhängigkeit vom Abstand von dieser Oberfläche.

Bei der katalytischen Umsetzung von mindestens zwei Reaktanten eines Strömungsmediums ist es zur Erreichung hoher Abscheidegrade erforderlich, die Reaktanten in einem geeigneten stöchiometrischen Verhältnis zueinander mit einem Katalysator in Kontakt zu bringen. Für die katalytische Umsetzung von in einem Ab- oder Rauchgas enthaltenen Stickoxiden nach dem Verfahren der selektiven katalytischen Reduktion (SCR) mit Ammoniak als Reduktionsmittel ist es beispielsweise erforderlich, daß Stickoxide und Ammoniak im zeitlichen Mittel zu etwa gleichen Teilen am Katalysator vorliegen. Die Einstellung des erforderlichen stöchiometrischen Verhältnisses von Stickoxiden zu Ammoniak oder einer in Ammoniak umwandelbaren Substanz, wie z. B. Harnstoff, ist nur zufriedenstellend durchführbar, wenn die Stickoxidkonzentration im Ab- oder Rauchgas gemessen oder über eine Kennfelddiagnose vergleichsweise genau bestimmt werden kann.

Die Messung der Stickoxidkonzentration erfordert jedoch einen vergleichsweise hohen Aufwand und verursacht vergleichsweise hohe Kosten. Solche Messungen können beispielsweise nur bei großen SCR-Anlagen in Kraftwerken durchgeführt werden, wobei jedoch auch hier ein lokaler Schlupf von Ammoniak in Folge von Stickoxid-Schieflagen nur unbefriedigend verhindert werden kann. Eine Vermeidung des Schlupfes von Ammoniak ist jedoch unter allen Umständen anzustreben, da Ammoniak giftig ist und bereits in äußerst geringer Konzentration eine Geruchsbelästigung für den Menschen darstellt (Geruchsschwelle etwa 5 ppm).

Weil sich die Stickoxidkonzentration in Rauchgasen von Kraftwerken, die mit fossilen Brennstoffen betrieben werden, aufgrund von sich zeitlich nur langsam vollziehenden Lastwechseln ebenfalls in zeitlichen Mittel entsprechend nur langsam ändern, wird eine Ammoniak-Dosierung anhand von aufwendigen Stickoxid- und ggfs. auch Ammoniak-Messungen, die relativ große Zeitkonstanten aufweisen, durchgeführt. Damit kann die Ammoniak-Dosierung für Rauchgase mit sich nur zeitlich langsam vollziehenden Änderungen der Stickoxidkonzentration als einigermaßen zufriedenstellend gelöst gelten.

Bedeutend schwieriger ist die Entstickung von Abgasen, die von Diesel- und Magermotoren emittiert werden. Ein für diese Motoren ausgelegtes System mit einem "Geregelten Dieselkatalysator" (GDK) muß aufgrund von unterschiedlichen Betriebszuständen und schnellen Lastwechseln dieser Motoren für die daraus resultierenden großen Schwankungen des Abgasvolumenstroms, der Abgastemperatur und der Stickoxidkonzentration im Abgas ausgelegt sein. Weil Ammoniak aufgrund seiner Gefährlichkeit nicht selbst in Fahrzeugen, wie z. B. PKW, Lastwagen, Omnibussen, Lokomotiven und Schiffen, mitgeführt werden darf, wird das notwendige Reduktionsmittel stattdessen beispielsweise in Form einer wäßrigen Harnstofflösung im Fahrzeug mitgeführt, aus der dann Ammoniak idealerweise in der augenblicklich gerade benötigten Menge erzeugt wird.

Weil der Einsatz der derzeit bekannten Stickoxidsensoren in Fahrzeugen aufgrund des hohen Aufwands und der hohen Kosten äußerst unwahrscheinlich ist, versucht man stattdessen, die pro Zeiteinheit vom Motor erzeugten Stickoxidmengen durch Kennfeldvergleich zu ermitteln, diesen daraus berechneten Stickoxidmengen entsprechend Reduktionsmittel zuzudosieren und die Abscheideleistung des Katalysators in einem On-Board-Diagnose-System zu simulieren (vergleiche beispielsweise die deutsche Patentanmeldung P 43 15 278.3). Somit gibt es für die Zudosierung des Reduktionsmittels bei dem GDK-System bislang keine geeignete Kontrollmöglichkeit, wodurch im Fall von Defekten am Katalysator oder am Dosierventil Belästigungen durch überhöhten Ammoniakschlupf auftreten können.

Der Erfindung liegt daher die Aufgabe zugrunde, einen Sensor anzugeben, der eine Einstellung der Konzentration von mindestens zwei Reaktanten eines Strömungsmediums in einem für eine katalytische Umsetzung dieser Reaktanten vorteilhaften stöchiometrischen Verhältnis zueinander unterstützt.

Diese Aufgabe wird erfindungsgemäß dadurch gelöst, daß ein Sensor zur Bestimmung des Verlaufs der Konzentration eines aus einem Strömungsmedium heraus durch eine Oberfläche eines Sensormaterials hindurch in das Sensormaterial eindiffundierenden, adsorbierbaren Stoffes, wie z. B. Ammoniak NH₃, in Abhängigkeit vom Abstand von dieser Oberfläche vorgesehen ist, bei dem in dem Sensormaterial und ggfs. auf der Oberfläche des Sensormaterials mit elektrischen Zuleitungen versehene Kontaktpaare vorgesehen sind, mittels derer die elektrische Leitfähigkeit des Sensormaterials zwischen den Kontakten der einzelnen Kontaktpaare bestimmbar ist.

Auf diese Weise kann, abhängig von der Tatsache, daß die spezifische Leitfähigkeit des Sensormaterials von der lokalen Konzentration des am Sensormaterial adsorbierten Stoffes abhängt, eine zuverlässige Aussage über den Verlauf der Konzentration des adsorbierten Stoffes in Abhängigkeit vom Abstand von der Materialoberfläche erstellt werden. Weil dieser adsorbierte Stoff zu der katalytischen Reaktion beiträgt und dabei verbraucht wird, wie z. B. Ammoniak bei der SCR-Reaktion, charakterisiert der an den einzelnen Kontaktpaaren gemessene elektrische Widerstand als Maß für die hervorgerufene chemische Reduzierung der Oberfläche in der jeweiligen Tiefenschicht des Sensormaterials indirekt die absolute Konzentration dieses Stoffes in dem Strömungsmedium. Dies wird erreicht, weil mit diesem Sensor aufzeigbar ist, welche Menge ein von außen in das Sensormaterial eindringender und an der katalytischen Umsetzung beteiligter zweiter Reaktant, wie z. B. Stickoxide, in den verschiedenen Tiefen des Sensors von den ursprünglich bis dahin vorgedrungen und dort adsorbierten Stoffmengen des ersten Reaktanten übrig ließen. Im besonderen bei der katalytischen Umsetzung von Stickoxiden mit Ammoniak kann mit dem Sensor die Dicke einer Reaktionszone bestimmt werden, wobei die Dicke der Reaktionszone als die Materialtiefe definiert ist, bei der der Tiefenverlauf der Konzentration des am Sensormaterial adsorbierten Ammoniaks ein lokales Minimum durchläuft, was im einzelnen anhand der Ausführungsbeispiele noch näher erläutert wird.

Zur Bestimmung des Tiefenverlaufes bildet man den Sensor vorteilhaft dahingehend aus, daß die Kontaktpaare mit dem zwischen den Kontakten befindlichen Sensormaterial in verschiedenen Abständen von der Oberfläche in einer Sensormaterialschicht angeordnet sind. Auf diese Weise kann die Konzentration eines Reaktanten in verschiedenen Abständen von der Oberfläche im Sensormaterial gemessen werden.

In vorteilhafter Ausgestaltung der Erfindung ist auf einer Trägerschicht eine Sensormaterialschicht angeordnet, in der die Kontaktpaare in Eindringrichtung des am Sensormaterial adsorbierbaren Stoffes in verschiedenen Abständen von der dem Strömungsmedium zugewandten Oberfläche angeordnet sind. Auf diese Weise können die Zuleitungen zu den Kontaktpaaren in vorteilhafter Weise durch die Trägerschicht geführt sein und mittels der Trägerschicht isoliert und abgestützt sein. Aufgrund einer auf diese Weise auch möglichen spaltenartigen Anordnung der Kontaktpaare ist es möglich, die Meßwerte der elektrischen Leitfähigkeit innerhalb einer Zeile von Kontaktpaaren und auch den Tiefenverlauf der elektrischen Leitfähigkeit ggfs. in benachbarten Spalten zu vergleichen, wodurch sich die Konzentration des adsorbierten Stoffes in Abhängigkeit von einem Tiefenbereich des Sensors ermitteln läßt. Eine Gleichspannung oder eine periodisch umgepolte Gleichspannung oder eine Wechselspannung kann dabei sukzessiv an die einzelnen Kontaktpaare angelegt werden oder aber auch kollektiv an allen Kontaktpaaren gleichzeitig anliegen. Der jeweils gemessene Gleichstrom- oder Wechselstromwiderstand oder Verlustwinkel kann zeitlich nacheinander über Meßstellenumschalter und Analog- Digital-Konverter erfaßt werden.

Zu einer genauen Messung der spezifischen Leitfähigkeit eines Tiefenbereiches ist es erforderlich, das Eindringen des am Sensormaterial adsorbierbaren Stoffes nur durch die dem Strömungsmedium zugewandte Oberfläche der Sensormaterialschicht zu erlauben. Dies wird erreicht, wenn die Abstände jedes Kontaktpaares zu den dem Strömungsmedium nicht zugewandten Oberflächen der Sensormaterialschicht größer sind als der Abstand des am weitesten von der dem Strömungsmedium zugewandten Oberfläche entfernten Kontaktpaares.

Alternativ dazu können die dem Strömungsmedium nicht zugewandten Oberflächen der Sensormaterialschicht gasdicht verschlossen sein, was eine Ausführung des Sensors mit besonders geringen Abmessungen erlaubt. Die dem Strömungsmedium nicht zugewandten Oberflächen sind dabei die Flächen, die nicht vom Strömungsmedium angeströmt werden.

Zu einer besonders genauen Messung der spezifischen Leitfähigkeit eines Tiefenbereichs und damit zur besonders genauen Bestimmung der Konzentration des in diesem Tiefenbereich adsorbierten Stoffes ist es vorteilhaft, wenn ein erster Abstand zwischen den beiden Kontakten eines Kontaktpaares kleiner ist als ein zweiter Abstand zwischen unmittelbar benachbart angeordneten Kontaktpaaren. Auf diese Weise können Spannungseinkopplungen benachbarter Kontaktpaare weitgehend unterdrückt werden.

In besonders vorteilhafter Ausgestaltung der Erfindung umfaßt der Sensor Mittel zur Temperierung der Sensormaterialschicht und/oder zur Messung der Temperatur der Sensormaterialschicht. Auf diese Weise können Korrekturen des Tiefenverlaufs der spezifischen Leitfähigkeit, die auf einem temperaturabhängigen Speichervermögen des Sensormaterials für den adsorbierbaren Stoff beruhen, weitgehend eleminiert werden.

Um den Sensor besonders sensitiv für ein Reduktionsmittel, wie z. B. Ammoniak, Harnstoff oder dergleichen, zu machen, ist es vorteilhaft, wenn das Sensormaterial ein Oxid mit einem pH-Wert kleiner als 7 umfaßt. Bei Oxiden mit diesen pH-Werten ändert sich die elektrische Leitfähigkeit des Sensormaterials innerhalb solcher Grenzen, daß eine Bestimmung der Konzentration des adsorbierten Reduktionsmittels aufgrund von Leitfähigkeitsunterschieden meßtechnisch einfach möglich ist.

Zum Einsatz des erfindungsgemäßen Sensors bei der katalytischen Umsetzung von Stickoxiden mit Ammoniak sieht eine besonders vorteilhafte Ausgestaltung der Erfindung vor, daß das Sensormaterial Titandioxid TiO₂ und einen oder mehrere der Bestandteile Wolframoxid WO₃, Molybdänoxid MoO₃, Vanadinpentoxid V₂O₅ und VₓMo_{y}O_{32-z} mit x+y ≦ 12; x, y ≧ 1 und z ≦ 1 umfaßt. Dieses Sensormaterial hat dieselben katalytischen Eigenschaften, wie das im Katalysator verwendete katalytisch aktive Material. Daher entspricht der Tiefenverlauf der Konzentration des am Sensormaterial adsorbierbaren Stoffes direkt dem Tiefenverlauf der Konzentration des am katalytisch aktiven Material adsorbierten Stoffes. Daher kann die unter Umständen sonst erforderliche tatächliche oder rechnerische Angleichung oder Adaption der Charakteristik des Sensormaterials an die Charakteristik des katalytisch aktiven Materials entfallen. Das trägt zu einer Steigerung der Dosiergenauigkeit bei.

Ausführungsbeispiele der Erfindung werden anhand der Zeichnung näher erläutert. Dabei zeigen:
- Figur 1: eine schaubildliche Ansicht der Vorstufe eines ersten Sensors;
- Figur 2: eine Vergrößerung des Ausschnitts II der Figur 1;
- Figur 3: eine schaubildliche Ansicht des fertigen ersten Sensors;
- Figur 4: in qualitativer Darstellung die Konzentration des am Sensormaterial adsorbierten Ammoniaks in Abhängigkeit vom Abstand von der dem Strömungsmedium zugewandten Oberfläche für verschiedene Zeitpunkte t₁ bis t₆;
- Figur 5: einen qualitativen Verlauf der Konzentration des am Sensormaterial adsorbierten Ammoniaks in Abhängigkeit vom Abstand von der dem Strömungsmedium zugewandten Oberfläche für verschiedene Ammoniak- und Stickoxid-Konzentrationen in einem Abgas.
- Figur 6: eine schaubildliche Ansicht eines für den Einsatz im Fahrzeug adaptierten zweiten Sensors;
- Figur 7: eine schematische Ansicht eines Kontaktpaares des Sensors der Figur 6; und
- Figur 8: einen Schnitt längs der Linie VIII-VIII der Figur 6.

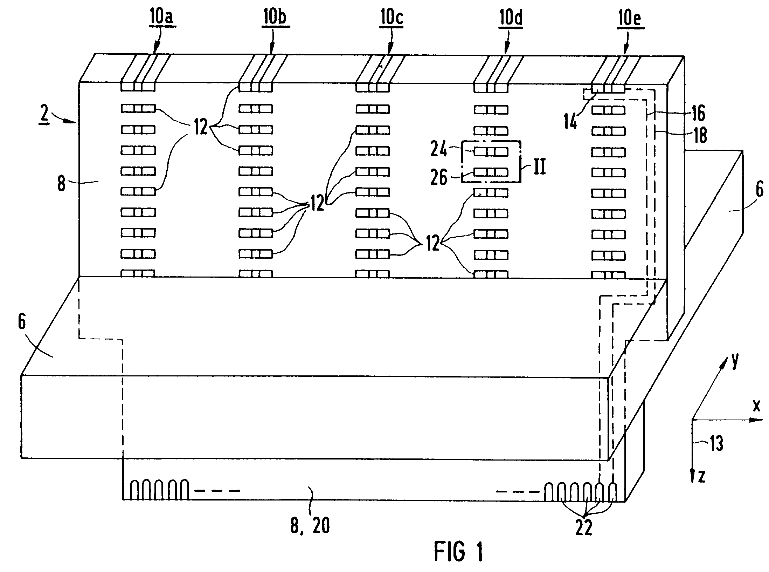

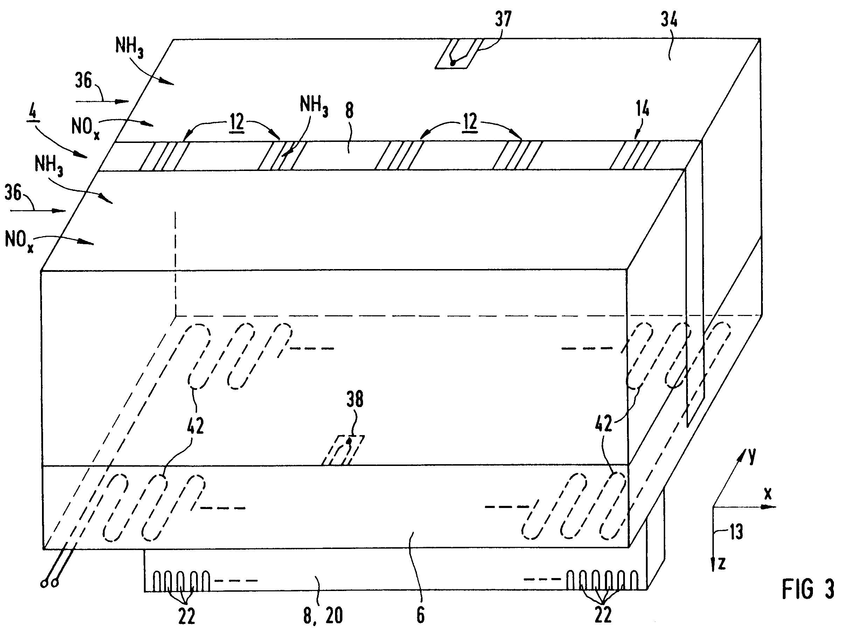

Eine in Figur 1 dargestellte Vorstufe 2 eines ersten Sensors 4 (vergleiche Figur 3) umfaßt eine Trägerschicht 6 aus Aluminiumoxid Al₂O₃ mit einer in einer xy-Ebene ausgerichteten Schichtebene. In diese Trägerschicht 6 ist eine weitere als Matrixplatine 8 bezeichnete Trägerschicht eingesetzt, die ebenfalls aus Aluminiumoxid Al₂O₃ besteht und parallel zu einer xz-Ebene ausgerichtet ist. In der Matrixplatine 8 sind oberhalb der Trägerschicht 6 fünf Spalten 10a bis 10e mit jeweils zehn Kontaktpaaren 12 angeordnet. Die Kontaktpaare 12 einer jeden Spalte 10a bis 10e sind übereinander parallel zu einer z-Richtung angeordnet. Diese z-Richtung entspricht einer Eindringrichtung 13 für einen aus einem Strömungsmedium heraus durch eine Oberfläche 33 eines Sensormaterials 34 (vergleiche Figur 3) in das Sensormaterial 34 eindiffundierenden, adsorbierbaren Stoff, wie z. B. Ammoniak NH₃.

An einem ausgewählten Kontaktpaar 14 sind elektrische Leitungen 16, 18 (gestrichelt dargestellt) angeschlossen, die innerhalb der Matrixplatine 8 verlaufen und unterhalb der Trägerschicht 6 in einem als Stecker 20 ausgebildeten Stück der Matrixplatine 8 in Kontaktzungen 22 enden. Die Kontaktzungen 22 sind hier nur in einem Teil des Steckers 20 eingezeichnet, sind aber bei einem fertigen Sensor 4 über die gesamte Breite des Steckers 20 beidseitig der Matrixplatine 8 in x-Richtung ausgedehnt. Der Stecker 20 kann auf einen hier nicht weiter dargestellten Sockel aufgesteckt werden, an dem die Kontaktpaare 12, 14 über die elektrischen Leitungen 16, 18 mit einer Gleichspannung oder einer periodisch umgepolten Gleichspannung oder einer Wechselspannung beaufschlagbar sind. Die Steckverbindung ist prinzipiell mit einer Steckverbindung vergleichbar, die beispielsweise bei Personal Computern zum Verbinden von Graphikkarten, Karten mit digitalen Signalprozessoren usw. mit dem Motherboard des Personal Computers verwendet werden.

Die in Figur 1 eingestrichelten elektrischen Leitungen 16, 18 verlaufen innerhalb der Matrixplatine 8 und sind gegeneinander und nach außen durch das Aluminiumoxid isoliert.

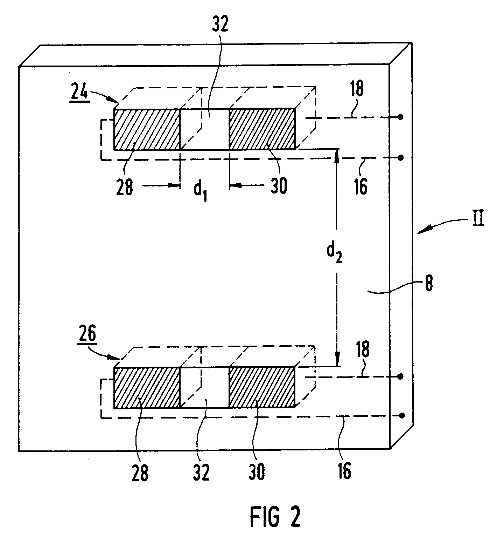

Der in Figur 2 perspektivisch und vergrößert dargestellte Ausschnitt II gemäß Figur 1 verdeutlicht den Aufbau ausgewählter Kontaktpaare 24, 26, die mit den übrigen Kontaktpaaren 12,14 identisch sind. Die Kontaktpaare 24, 26 umfassen jeweils zwei metallische Kontakte 28, 30, zwischen denen ein Durchgriff 32 zur Aufnahme von Sensormaterial vorgesehen ist. Mittels der Kontaktpaare 24, 26 ist über ihre elektrischen Zuleitungen 16, 18 die Spannung meßbar, die über den würfelartigen Durchgriff 32 mit der Kantenlänge d₁ des Durchgriffes 32 in dem Sensormaterial abfällt. Ein Abstand d₂ zwischen zwei unmittelbar übereinander angeordneten Kontaktpaaren 24, 26 ist erheblich größer als der Abstand d₁, über den die am Sensormaterial abfallende Spannung mittels der Kontakte 28, 30 für jedes Kontaktpaar 12, 14, 24, 26 gemessen wird. Damit ist gewährleistet, daß die gemessenen Werte für die spezifische Leitfähigkeit einem definierten und räumlich beschränkten Tiefenbereich des Sensormaterials zuweisbar sind. Störungen der Leitfähigkeitsmessung durch Einkopplung von Spannungen anderer, benachbarter Kontaktpaare sind auf diese Weise weitgehend unterdrückt.

In Figur 3 ist der fertige erste Sensor 4 mit der auf der Trägerschicht 6 aufgebrachten Sensormaterialschicht 34 dargestellt. Im hier vorliegenden Fall eines mit Stickoxiden NOₓ und Ammoniak NH₃ beladenen Abgases 36 umfaßt die Sensormaterialschicht 34 bekanntes DeNOₓ-Material, das im Ausführungsbeispiel Titandioxid und einen oder mehrere der Bestandteile Wolframoxid, Molybdänoxid, Vanadinpentoxid und VₓMo_{y}O_{32-z} mit x+y ≦ 12, x, y ≧ 1 und z ≦ 1 umfaßt. Außerdem umfaßt der fertige Sensor 4 auf der dem Abgas 36 gewandten Oberfläche 33 ein Thermoelement 37 mit nicht weiter dargestellten Anschlüssen. Auf der hierzu gegenüberliegenden Oberfläche, d. h. zwischen der Trägerschicht 6 und der Sensormaterialschicht 34, ist ein weiteres Thermoelement 38 zur Temperaturerfassung angeordnet. Als Thermoelemente 37, 38 können beispielsweise Nickelchrom/Nickel-Thermoelemente verwendet werden, deren Thermospannungen an hier nicht weiter dargestellten Kontakten abgegriffen werden können. Mittels der Thermoelemente 37, 38 ist es möglich, Mittel 42 zur Temperierung des Sensors 4, hier vorliegend als auf die Unterseite der Trägerschicht 6 aufgeklebte Heizmäander 42 (ausschnittweise gestrichelt eingezeichnet), einzustellen. Im Ausführungsbeispiel wird der Sensor 4 auf etwa 400 °C temperiert, wodurch temperaturabhängige Veränderungen der Adsorptionscharakteristik der Sensormaterialschicht 34 eleminiert werden. Der auf diese Weise erhaltene feste Temperaturbezugspunkt trägt dazu bei, daß die mittels des Sensors 4 erhaltenen Leitfähigkeitswerte sowie die daraus resultierenden Werte für die Konzentration des adsorbierten Ammoniaks reproduzierbar sind. Bei Kenntnis der Adsorptionscharakteristik der Sensormaterialschicht 34 in Abhängigkeit von der Temperatur T sind die gewonnenen Meßergebnisse auf einen mit unterschiedlichen Temperaturen betriebenen Katalysator anpaßbar.

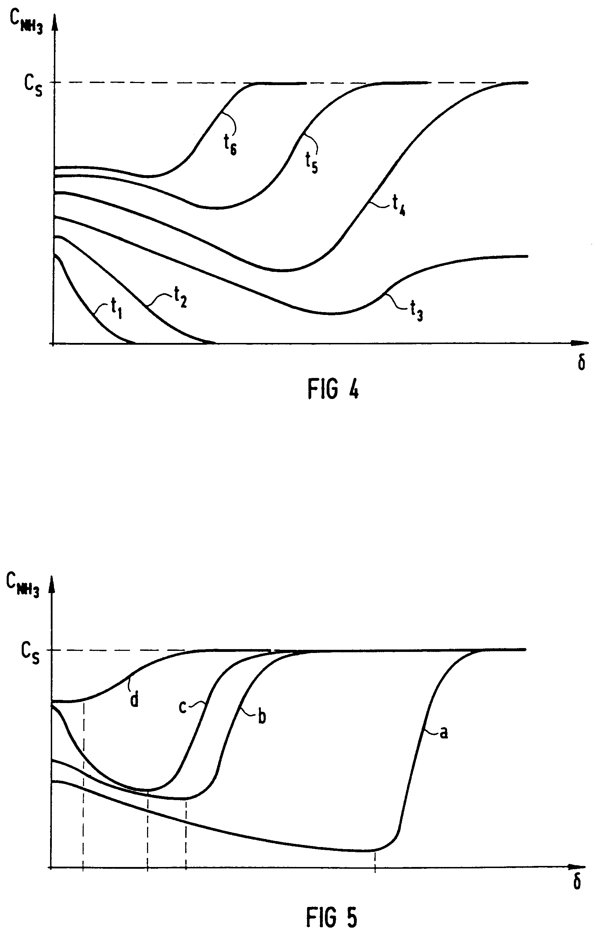

In Figur 4 sind für Zeiten t₁ bis t₆ die mittels des Sensors 4 gemessenen Verläufe der Konzentration des im Sensormaterial 34 adsorbiertem Ammoniaks in Abhängigkeit von der Eindringtiefe δ im Sensormaterial 34 dargestellt. Dabei wird die Eindringtiefe δ in der positiven z-Richtung der Figuren 1 und 3 gemessen. Auf der linken Seite des Diagramms, bei δ = 0, sollte daher die gemessene Konzentration stets gleich derjenigen im angrenzenden Gasraum sein. Der zum Zeitpunkt t₀ ammoniakfreie Sensor 4 füllt sich zunächst nur relativ langsam mit aus dem Abgas 36 adsorbierten Ammoniak (vergleiche Kurven zu den Zeiten t₁ und t₂). Zum Zeitpunkt t₃ wird im Sensormaterial 34 mehr Ammoniak adsorbiert als durch die katalytische Reduktion von Stickoxiden mit Ammoniak verbraucht wird, wodurch sich ein lokales Minimum der Konzentration von adsorbiertem Ammoniak bei einer Eindringtiefe von etwa 100 µm ergibt. Aufgrund einer weiteren die Konzentration von Stickoxiden im Abgas 36 übersteigenden Ammoniakzudosierung bis jeweils zu den Zeitpunkten t₄, t₅ und t₆ steigen die im Sensormaterial 34 hinter einer Reaktionszone (vergleiche lokale Minima) adsorbierten Ammoniakmengen bis auf einen Sättigungswert Cₛ an, der bei dem auf 400 °C temperierten Sensormaterial 34 bei etwa 10¹⁹ Ammoniakmolekülen pro cm³ liegt. Eine weiterhin gleichbleibende und über den Zeitpunkt t₆ hinausgehende Zudosierung von Ammoniak würde ein "Überlaufen" des Sensor 4 verursachen, d. h. es würde kein Ammoniak mehr im Katalysator adsorbiert und folglich überschüssiger Ammoniak aus dem Katalysator austreten. Dies würde einen beim GDK-Einsatz unbedingt zu vermeidenden Ammoniakschlupf zur Folge haben. Dieser Schlupf steigt auch an, wenn insbesondere bei steigenden Abgastemperaturen die Temperatur eines beispielsweise aus dem Sensormaterial 34 bestehenden und weitgehend mit Ammoniak gefüllten Katalysators ansteigt. Die spezifische Adsorptionsfähigkeit des Sensormaterials 34 für Ammoniak nimmt mit steigender Temperatur ab, weshalb der Katalysator beim GDK-Einsatz nie bis zur Kapazitätsgrenze mit Ammoniak aufgefüllt werden sollte.

Anhand des Verlaufes der Dicke der Reaktionszone ist eine Bestimmung der Stickoxidkonzentration im Abgas 36 sowie eine daran angepaßte Ammoniakzudosierung möglich.

Figur 5 zeigt einige typische Verläufe der Konzentration des an einem Katalysatormaterial adsorbierten Ammoniaks in Abhängigkeit von der Eindringtiefe δ für verschiedene Betriebszustände a bis d eines Dieselmotors. Diese Verläufe sind durch Messung des Verlaufs der Konzentration an im Sensormaterial 34 adsorbierten Ammoniaks des Sensors 4 bestimmt und interpoliert worden.

Der Betriebszustand a zeichnet sich durch eine länger andauernde stark unterstöchiometrische Zudosierung von Ammoniak NH₃ in bezug zu den im Rauchgas 36 enthaltenen Stickoxiden NOₓ aus. Bis zur vollständigen Abreaktion der Stickoxide NOₓ müßten diese relativ weit in das Katalysatormaterial eindringen. Die Dicke der Reaktionszone liegt hier nur wenig unterhalb 200 µm. Da viele Stickoxidmoleküle in den oberen Bereichen (δ ≈ 50 µm) des Katalysatormaterials keinen Reaktionspartner finden, wird bei diesem Betriebszustand ein relativ hoher Stickoxidschlupf erhalten.

Zur Verringerung dieses Schlupfes trägt eine Steigerung der Ammoniakzudosierung bei, so daß sich bei etwa stöchiometrischer Zudosierung von Ammoniak Betriebszustände mit den zugehörigen Kurvenverläufen b und c ergeben. Die sich bei diesen Betriebszuständen einstellende Schichtdicke der Reaktionszone zwischen etwa 50 und 80 µm ist in diesem Ausführungsbeispiel vorteilhafter gegenüber den Kurvenverläufen a und d sowie deren Betriebscharakteristik. Bei dem Betriebszustand d überwiegt die in das Abgas 36 eingebrachte Ammoniakkonzentration die im Abgas 36 vorherrschende Stickoxidkonzentration. Die Dicke der Reaktionszone geht relativ stark zurück, so daß innerhalb kurzer Zeit das Adsorptionsvermögen des katalytisch aktiven Materials für Ammoniak NH₃ erschöpft ist. Infolgedessen tritt nicht-adsorbierter und nicht bei der Reduktion verbrauchter Ammoniak als Schlupf auf. Die Zudosierung von Ammoniak ist daher bei Auftreten dieses Betriebszustandes zurückzunehmen, wodurch sich der Kurvenverlauf d wieder an die Kurvenverläufe b, c annähert.

Die in den Figuren 4 und 5 angegebenen Eindringtiefen und Dicken der Reaktionszone sind nicht repräsentativ. Vielmehr hängen sie empfindlich von der chemischen Zusammensetzung und Porenstruktur des Sensormaterials 34, der Temperatur des Abgases 36 und des Sensormaterials 34, von der Strömungsgeschwindigkeit des Abgases 36 sowie von der Art der Anströmung (laminar/turbulent) ab. Die Adsorptionscharakteristik des Sensormaterials 34 und des katalytisch aktiven Materials wird daher individuell auf die gegebenen Abhängigkeiten hin untersucht. Die Ergebnisse dieser Untersuchung werden beispielsweise in Kennfeldern abgelegt. Ein in einem Fahrzeug mitgeführtes On-Board-Diagnosesystem kann diese Kennfelddaten abrufen und daraus eine Zudosierung des Ammoniaks, beispielsweise in Form einer wässrigen Harnstofflösung, ableiten, so daß hohe Abscheidegrade für die im Abgas 36 enthaltenen Stickoxide bei gleichzeitig vernachlässigbarer Ammoniakschlupf erreichbar sind.

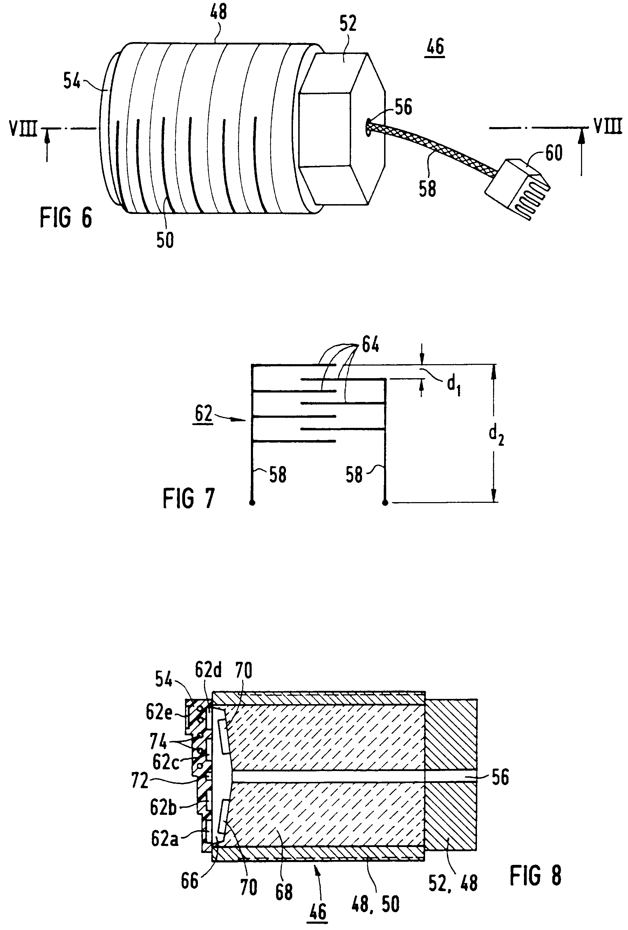

Figur 6 zeigt einen für den Einsatz im Fahrzeug adaptierten, zweiten Sensor 46, der ähnlich wie eine von der Automobiltechnik her bekannte Zündkerze aufgebaut ist. Der Sensor 46 umfaßt einen becherförmigen Metallkörper 48, in dessen Außenumfang ein Gewinde 50 eingedreht ist. Der Becherboden des Metallkörpers 48 ist als Sechskant 52 ausgeführt. Dieser Sechskant 52 bietet eine Angriffsfläche für ein Werkzeug, mit dem der Sensor 46 beispielsweise in einen DeNOₓ-Katalysator mit entsprechender Gewindebohrung einschraubbar ist.

Die Öffnung des becherförmigen Metallkörpers 48 wird mit einer auf den Metallkörper 48 aufgesetzten Scheibe 66, die als Trägerschicht dient, und darauf aufgebrachtem Sensormaterial 54 mit darin zur Leitfähigkeitsmessung integrierten Kontaktpaaren 62a bis 62e (vergleiche Figur 8) verschlossen. Außerdem sind in der Scheibe 66 mit Sensormaterial 54 neben den Kontaktpaaren (62a bis 62e) auch ein PT100-Widerstand (72) zur Temperaturmessung sowie kleine Heizmäander 70 zur Temperierung des Sensormaterials 54 integriert, was noch anhand von Figur 8 näher gezeigt wird. Im Inneren ist der Becher mit einer keramischen Masse 68 (vergleiche Figur 8), wie z. B. Aluminiumoxid aufgefüllt, die aber ebenso wie der Sechskant 52 eine zentrale Bohrung 56 für elektrische Zuleitungen 58 der in dem Sensormaterial 54 integrierten Komponenten hat. Zur Widerstandsmessung werden die Zuleitungen 58 über einen Stecker 60 an eine nicht weiter dargestellte Auswertelektronik angeschlossen.

In Figur 7 ist eines der im Sensormaterial 54 integrierten Kontaktpaare 62a bis 62e schematisch dargestellt. Wie aus Figur 7 ersichtlich ist, ist ein Abstand d1 zwischen zwei unmittelbar benachbarten Leitern 64 erheblich kleiner als ein Abstand d2 zwischen benachbarten Kontaktpaaren 62. Auf diese Weise kann der integral über dem Kontaktpaar 62 abfallende Widerstand ohne Spannungseinkopplungen benachbarter Kontaktpaare 62 gemessen werden.

In dem Schnittbild entlang der Linie VIII-VIII der Figur 6 - nachfolgend in Figur 8 dargestellt - erkennt man die Anordnung der einzelnen Kontaktpaare 62a bis 62e. Die Kontaktpaare 62a bis 62d sind auf die flache Aluminiumoxidscheibe 66 aufgebracht. Diese Scheibe 66 verschließt die Öffnung des becherförmigen Metallkörpers 48. Unterhalb der Scheibe 66 sind auf der keramischen Masse 68 aus Aluminiumoxid Heizmäander 70 zur Temperierung des Sensormaterials 54 vorgesehen. Auf eine Darstellung der elektrischen Zuleitungen 58 der Figur 6 zu den Kontaktpaaren 62a bis 62e, den Heizmäandern 70 und einem PT100-Widerstand 72 zur Temperaturmessung des Sensormaterials 54 ist verzichtet worden.

In Abweichung von dem Konzept, das dem Sensor 4 der Figur 1 und Figur 3 zugrundeliegt, sind hier die einzelnen Kontaktpaare 62a bis 62e unterschiedlich dick mit dem beispielsweise von der Zusammensetzung her aus Figur 3 bekannten Sensormaterial 34, hier 54, beschichtet. Über den Kontaktpaaren 62c und 62d ist darüber hinaus in dem Sensormaterial 54 eine Armierung aus Glasfasern 74 vorgesehen, die zur Haftverbesserung des Sensormaterials 54 auf der Aluminiumoxidscheibe 66 beiträgt.

Der gegenüber dem Sensor 4 der Figur 3 erheblich einfacher aufgebaute und erheblich einfacher zu produzierende Sensor 46 arbeitet natürlich nach demselben Prinzip wie der Sensor 4. Aufgrund der unterschiedlich hohen Beschichtungsdicke ist auch hier wieder eine Aussage über die adsorbierte Menge eines aus einem Strömungsmedium heraus an dem Sensormaterial 54 adsorbierten Stoffes in der jeweiligen Tiefenschicht möglich.

Darüber hinaus kann auch bei diesem Sensor 46 mittels des Kontaktpaares 62e nachvollzogen werden, welche Mengen des adsorbierten Stoffes, also beispielsweise Ammoniak, nach einem impulsartigen Aufheizen des Sensormaterials 54 mittels der Heizvorrichtungen 70 an der Oberfläche nach dem Durchlaufen einer bestimmten definierten Schichtdicke ankommen. Somit gewährleistet auch dieser Sensor 46 bei entsprechend nachgeschalteten Auswertemitteln, wie z. B. einem On-Board-Diagnose-System, eine weitgehende katalytische Umsetzung der Stickoxide bei vernachlässigbaren Ammoniakschlupf.

Die vorgestellten Sensoren 4, 46 eignen sich aber ebenso für alle anderen katalytischen Prozesse, bei denen mindestens ein Reaktant eines flüssigen oder gasförmigen Strömungsmediums in adsorbierter Form am Katalysator vorliegen muß.

## Patentansprüche

1. Sensor (4, 46) zur Bestimmung des Verlaufs der Konzentration eines aus einem Strömungsmedium (36) heraus durch eine Oberfläche (33) eines Sensormaterials (34, 54) hindurch in das Sensormaterial (34, 54) eindiffundierenden, adsorbierbaren Stoffes, wie z. B. Ammoniak NH₃, in Abhängigkeit vom Abstand von dieser Oberfläche (33), bei dem in dem Sensormaterial (34, 54) und ggfs. auf der Oberfläche des Sensormaterials (34, 54) mit elektrischen Zuleitungen (16, 18, 58) versehene Kontaktpaare (12, 62) vorgesehen sind, mittels derer die elektrische Leitfähigkeit des Sensormaterials (34, 54) zwischen den Kontakten (28, 30,62) der einzelnen Kontaktpaare (12) bestimmbar ist.

2. Sensor nach Anspruch 1,
**dadurch gekennzeichnet**, daß die Kontaktpaare (12, 62) mit dem zwischen den Kontakten (28, 30) befindlichen Sensormaterial (34, 54) in verschiedenen Abständen von der Oberfläche (33) in einer Sensormaterialschicht (34, 54) angeordnet sind.

3. Sensor nach Anspruch 1 oder 2,
**dadurch gekennzeichnet**, daß auf einer Trägerschicht (6, 8) eine Sensormaterialschicht (34) angeordnet ist, in der die Kontaktpaare (12) in verschiedenen Abständen von der dem Strömungsmedium zugewandten Oberfläche (33) angeordnet sind.

4. Sensor nach Anspruch 3,
**dadurch gekennzeichnet**, daß die Abstände jedes Kontaktpaares (12, 62) zu den dem Strömungsmedium (36) nicht zugewandten Oberflächen der Sensormaterialschicht (34) größer sind als der Abstand des am weitesten von der dem Strömungsmedium (36) zugewandten Oberfläche (33) entfernten Kontaktpaares.

5. Sensor nach Anspruch 3,
**dadurch gekennzeichnet**, daß die dem Strömungsmedium (36) nicht zugewandten Oberflächen der Sensormaterialschicht (34) gasdicht verschlossen sind.

6. Sensor nach Anspruch 1 oder 2,
**dadurch gekennzeichnet**, daß auf einer Trägerschicht (66) mehrere Kontaktpaare (62a bis 62d) angeordnet sind, die unterschiedlich dick mit Sensormaterial (54) beschichtet sind.

7. Sensor nach einem der Ansprüche 1 bis 6,
**dadurch gekennzeichnet**, daß ein erster Abstand (d₁) zwischen den Kontakten (28, 30) eines Kontaktpaares (12, 24, 26, 62) kleiner ist als ein zweiter Abstand (d₂) zwischen unmittelbar benachbart angeordneten Kontaktpaaren (24, 26, 62a bis 62d).

8. Sensor nach einem der Ansprüche 3 bis 7,
**dadurch gekennzeichnet**, daß die Trägerschicht (6, 8, 66) die elektrischen Zuleitungen (16, 18, 58) für das Sensormaterial (34, 54) enthält.

9. Sensor nach einem der Ansprüche 3 bis 8,
**dadurch gekennzeichnet**, daß die Trägerschicht (66) und ggfs. eine darauf angebrachte Schicht aus Sensormaterial (54) von einer zylindrischen Hülse (48) mit einem Außengewinde (50) umgeben ist.

10. Sensor nach Anspruch 9,
**dadurch gekennzeichnet**, daß die Hülse (48) becherförmig ausgeführt ist, daß am Becherboden eine mehrkantige Angriffsfläche (52) für ein Werkzeug vorgesehen ist, und daß die elektrischen Zuleitungen (58) zu den Kontaktpaaren (62a bis 62e) durch den Becherboden geführt sind.

11. Sensor nach einem der Ansprüche 1 bis 10,
**dadurch gekennzeichnet**, daß Mittel zur Temperierung (42, 70) der Sensormaterialschicht (34, 54) vorgesehen sind.

12. Sensor nach einem der Ansprüche 1 bis 11,
**dadurch gekennzeichnet**, daß Mittel zur Messung der Temperatur (37, 38, 72) der Sensormaterialschicht (34, 54) vorgesehen sind.

13. Sensor nach einem der Ansprüche 1 bis 12,
**dadurch gekennzeichnet**, daß das Sensormaterial (34, 54) ein Oxid mit einem pH-Wert kleiner als 7 umfaßt.

14. Sensor nach einem der Ansprüche 1 bis 13,
**dadurch gekennzeichnet**, daß das Sensormaterial (34, 54) Titandioxid TiO₂ und einen oder mehrere der Bestandteile Wolframoxid WO₃, Molybdänoxid MoO₃, Vanadinpentoxid V₂O₅ und VₓMo_{y}O_{32-z} mit x+y ≦ 12; x, y, ≧ 1 und z≦1 umfaßt.
